# EUROPEAN PATENT SPECIFICATION

(11) **EP 1 418 950 B1**
(45) Date of publication and mention of the grant of the patent: **27.02.2008**
(21) Application number: 02740868.1
(22) Date of filing: 18.06.2002
(51) Int. Cl.: A61K 51/00, C07H 5/02

(54) **SOLID-PHASE NUCLEOPHILIC FLUORINATION**
NUCLEOPHILE FLUORIERUNG AN FESTER PHASE
FLUORATION NUCLEOPHILE EN PHASE SOLIDE

(30) Priority: 29.06.2001 GB 0115927
(43) Date of publication of application: 19.05.2004
(73) Proprietor: GE Healthcare Limited, Little Chalfont Buckinghamshire HP7 9NA (GB); Hammersmith Imanet Limited, London W12 0NN (GB)
(72) Inventor: LUTHRA, Sajinder, Kaur, Imaging Res. Solutions Ltd, London W12 0NN (GB); BRADY, Frank, London ,SW8 2AB (GB); WADSWORTH, Harry, John, Amersham Health plc, Amersham, Buckinghamshire HP7 9LL (GB); GIBSON, Alexander, Mark, Amersham Health plc, Amersham, Buckinghamshire HP7 9LL (GB); GLASER, Matthias, E., Imaging Res. Solutions Ltd., London W12 0NN (GB)
(74) Representative: Hammett, Audrey Grace Campbell
(86) International application number: PCT/GB2002/002505
(87) International publication number: WO 2003/002157

(56) References cited:
- WO-A-00/10614
- WO-A-99/23104
- US-A- 5 510 522
- TOORONGIAN S A ET AL: "ROUTINE PRODUCTION OF 2-DEOXY-2-U18FFLUORO-D-GLUCOSE BY DIRECT NUCLEOPHILIC EXCHANGE ON A QUATERNARY 4-AMINOPYRIDINIUM RESIN" INTERNATIONAL JOURNAL OF RADIATION APPLICATIONS AND INSTRUMENTATION PART B: NUCLEAR MEDICINE AND BIOLOGY, ELSEVIER SCIENCE PUBLISHERS, NEW YORK, NY, US, vol. 17, no. 3, 1990, pages 273-279, XP000676335 ISSN: 0883-2897
- HARADAHIRA T ET AL: "A NEW SYNTHESIS OF 2 DEOXY-2-(18F) FLUORO-D-GALACTOSE USING (18F) FLUORIDE ION" JOURNAL OF LABELLED COMPOUNDS AND RADIOPHARMACEUTICALS, SUSSEX, GB, vol. 25, no. 7, 1988, pages 721-729, XP000675934 ISSN: 0362-4803

## Description

The present invention relates to novel solid-phase processes for the production of radiolabelled tracers, in particular for the production of ¹⁸F-labelled compounds which may be suitable for use as Positron Emission Tomography (PET) radiotracers. The invention also comprises radiopharmaceutical kits using these novel processes.

The favoured radioisotope for PET, ¹⁸F, has a relatively short half-life of 110 minutes. ¹⁸F-labelled tracers for PET therefore have to be synthesised and purified as rapidly as possible, and ideally within one hour of clinical use. Standard synthetic methods for introducing fluorine-18 are relatively slow and require post-reaction purification (for example, by HPLC) which means that it is difficult to obtain the ¹⁸F-labelled tracer for clinical use in good radiochemical yield. There is also a need for automation to protect the operator from radiation exposure. Many radiofluorinations are complicated procedures and it is necessary to simplify them to facilitate automation.

International Journal Of Radiation Applications And Instrumentation Part B: Nuclear Medicine And Biology, Elsevier Science Publishers, New York, NY, US (1990), 17(3), 273-279 describes resin-bound ¹⁸F⁻ and its use to prepare [¹⁸F] FDG.

The present invention provides solid-phase processes for producing ¹⁸F-labelled tracers quickly and with high specific activity yet avoiding time-consuming purification steps, such that the resultant ¹⁸F-labelled tracer is suitable for use in PET. The solid-phase methods also lend themselves to automation with advantages of ease of production and greater throughput. The invention also comprises radiopharmaceutical kits which use such processes and thus provide the radiopharmacist or clinician with a convenient means of preparing an ¹⁸F-labelled tracer.

In a general aspect, the invention provides a process for the production of an ¹⁸F-labelled tracer which comprises treatment of a resin-bound precursor of formula

SOLID SUPPORT-LINKER- X -TRACER precursor (I)

with ¹⁸F⁻ to produce the labelled tracer of formula (II)

¹⁸F-TRACER (II)

As the ¹⁸F-labelled tracer of formula (II) is removed from the solid-phase into solution, all unreacted precursor remains bound to the resin and can be separated by simple filtration, thus obviating the need for complicated purification, for example by HPLC. The ¹⁸F-labelled tracer of formula (II) may be cleaned up by removal of excess F⁻, for example by ion-exchange chromatography and/or by removal of any organic solvent. The resultant ¹⁸F-labelled tracer of formula (II) may then be further made-up into an aqueous formulation for clinical use.

Examples of tracers which may be ¹⁸F-labelled in the manner of the invention include 2-fluoro-2-deoxy-D-glucose (FDG), 6-fluoro-L-DOPA (FDOPA), 3'-deoxy-3'-fluorothymidine (FLT), 2-(1,1-dicyanopropen-2-yl)-6-(2-fluoroethyl)-methylamino)-naphthalene (FDDNP), 2-, 5-, and 6-fluoro (2(S)-azetinylmethoxy)pyridines, N-succinimidyl-4-[18F]fluorobenzoate ([18F]-SFB) and peptides. In preferred aspects of the invention, the tracer produced is selected from FDG, FDOPA, FLT, and FDDNP, and is most preferably FDG or FDOPA.

In the compounds of formula (1), X is a group which promotes nucleophilic substitution at a specific site on the attached TRACER. Examples of X include -SO₂O- as in formula (Ia) below, I⁺ as in formula (Id) below, or -N(C₁₋₆alkyl)₂⁺- as in formula (If) below.

In a further aspect, the invention provides a process for the production of an ¹⁸F-labelled tracer which comprises treatment of a resin-bound precursor of formula (Ia)

SOLID SUPPORT-LlNKER-SO₂-O -TRACER PRECURSOR (Ia)

with ¹⁸F⁻ to produce the labelled tracer of formula (II)
¹⁸F-TRACER (II)
followed by optionally
   (i) removal of excess ¹⁸F⁻, for example by ion-exchange chromatography; and/or
   (ii) removal of any protecting groups; and/or
   (iii) removal of organic solvent; and/or
   (iv) formulation of the resultant compound of formula (II) as an aqueous solution.

In the compound of formula (Ia), the TRACER precursor is suitably FDG, FLT, FDDNP or a precursor thereof in which one or more functional groups have been protected, or an activated precursor of FDOPA. Most suitably, the TRACER precursor in the compound of formula (Ia) is FDG or a precursor thereof.

As shown in Scheme 1, the compound of formula (Ia) may be conveniently prepared from any sulphonic acid functionalised commercially available resin, such as Merrifield Resin, NovaSyn^{®} TG Bromo Resin, (Bromomethyl)phenoxymethyl polystyrene, or Wang Resin which may be reacted with a chlorinating agent to give the corresponding sulphonyl chloride resin. This may be carried out by treating the resin with, for example, phosphorus pentachloride, phosphorus trichloride, oxalyl chloride, or thionyl chloride, in an appropriate inert solvent such as dichloromethane, chloroform, or acetonitrile, and heating at elevated temperature for a period of time. The excess reagent may then be removed from the resin by washing with further portions of the inert solvent. The sulphonyl chloride resin may then be reacted with the alcohol analogue of the tracer to produce the resin-bound precursor of formula (Ia). This may be carried out by treating the resin with a solution of the alcohol in an inert solvent such as chloroform, dichloromethane, acetonitrile, or tetrahydrofuran containing a non-nucleophilic soluble base such as sodium hydride or a trialkylamine, for example triethylamine or diisopropylethylamine. The reaction may be carried out at a temperature of 10 to 80°C, optimally at ambient temperature for a period of from around 1 to 24 hours. The excess alcohol and base may then be removed from the solid support by washing with further portions of an inert solvent such as chloroform, dichloromethane, or tetrahydrofuran.

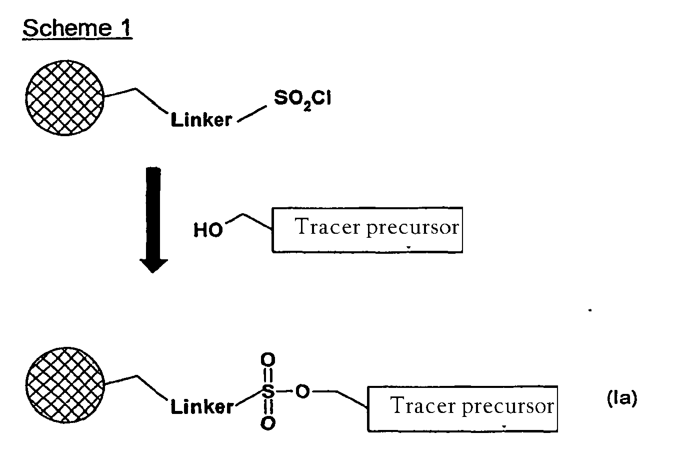

In the compounds of formulae (I) and (Ia) and in the following more specific aspects of the invention, the "SOLID SUPPORT" may be any suitable solid-phase support which is insoluble in any solvents to be used in the process but to which the LINKER and/or TRACER precursor can be covalently bound. Examples of suitable SOLID SUPPORT include polymers such as polystyrene (which may be block grafted, for example with polyethylene glycol), polyacrylamide, or polypropylene or glass or silicon coated with such a polymer. The solid support may be in the form of small discrete particles such as beads or pins, or as a coating on the inner surface of a cartridge or on a microfabricated vessel.

In the compounds of formulae (I) and (Ia) and in the following more specific aspects of the invention, the "LINKER" may be any suitable organic group which serves to space the reactive site sufficiently from the solid support structure so as to maximise reactivity. Suitably, the LINKER comprises zero to four aryl groups (suitably phenyl) and/or a C₁₋₆ alkyl or C₁₋₆haloalkyl (suitably C₁₋₆ fluoroalkyl), and optionally one to four additional functional groups such as amide or sulphonamide groups. Examples of such linkers are well known to those skilled in the art of solid-phase chemistry, but include: 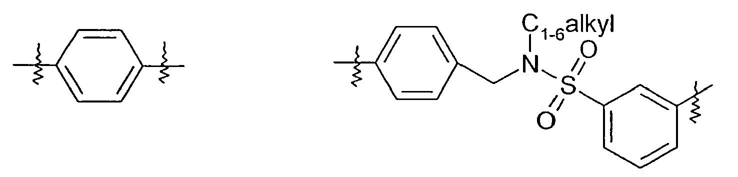 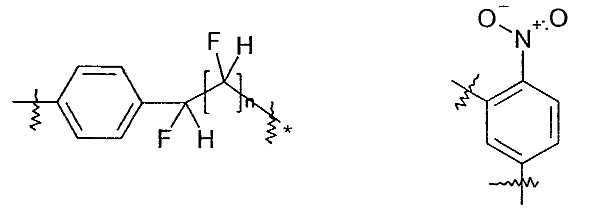 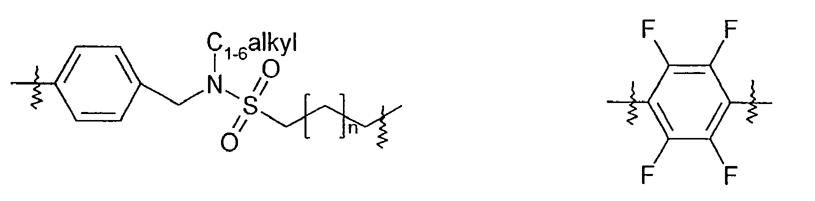 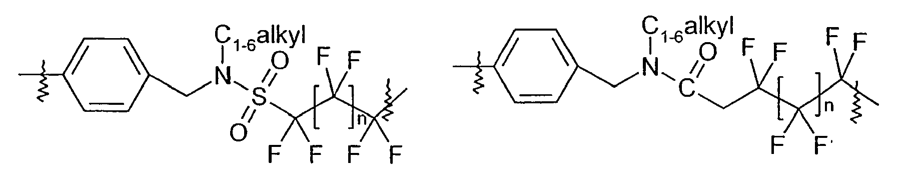 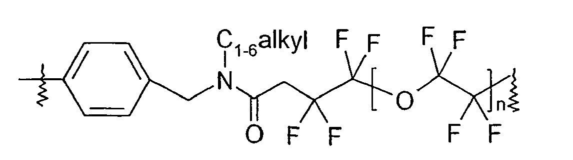 wherein at each occurrence, n is an integer of 0 to 3.

As would be apparent to the person skilled in the art it may be necessary to protect functional groups in the TRACER precursor to avoid unwanted reactions during the radiolabelling process. Such protection may be achieved using standard methods of protecting group chemistry. After the radiolabelling is complete, any protecting groups may be removed by simple procedures which are also standard in the art. Suitable protection and deprotection methodologies may be found, for example, in Protecting Groups in Organic Synthesis, Theodora W. Greene and Peter G. M. Wuts, published by John Wiley & Sons Inc.

Treatment of the compound of formula (I) or (Ia) with ¹⁸F⁻ may be effected by treatment with any suitable source of ¹⁸F⁻, such as Na¹⁸F, K¹⁸F, CS¹⁸F, tetraalkylammonium ¹⁸F fluoride, or tetraalkylphosphonium ¹⁸F fluoride. To increase the reactivity of the fluoride, a phase transfer catalyst such as 4,7,13,16,21,24 hexaoxa-1,10-diazabicyclo[8,8,8] hexacosane may be added and the reaction performed in a non protic solvent. These conditions give reactive fluoride ions. The treatment with ¹⁸F⁻ is suitably effected in the presence of a suitable organic solvent such as acetonitrile, dimethylformamide, dimethylsulphoxide, tetrahydrofuran, dioxan, 1,2 dimethoxyethane, sulpholane, N-methylpyrolidinineone, at a non-extreme temperature, for example, 15°C to 180°C, preferably at elevated temperature. On completion of the reaction, the ¹⁸F-labelled tracer of formula (II) dissolved in the solvent is conveniently separated from the solid-phase by filtration. The same fluorination techniques may be used in the following more specific aspects of the invention.

Any excess ¹⁸F⁻ may be removed from the solution of ¹⁸F-tracer by any suitable means, for example by ion-exchange chromatography or solid phase absorbents. Suitable ion-exchange resins include BIO-RAD AG 1-X8 or Waters QMA and suitable solid phase absorbents include alumina. The excess ¹⁸F⁻ may be removed using such solid phases at room temperature in aprotic solvents.

Any organic solvent may be removed by any standard method such as by evaporation at elevated temperature *in vacuo* or by passing a stream of inert gas such as nitrogen or argon over the solution.

Before use of the ¹⁸F-labelled tracer, it may be appropriate to formulate it, for example as an aqueous solution by dissolving the ¹⁸F-labelled tracer in sterile isotonic saline which may contain up to 10% of a suitable organic solvent such as ethanol, or a suitable buffered solution such as phosphate buffer. Other additives may be added such as ascorbic acid to reduce radiolysis.

The present invention provides, in a further aspect, a process for the production of 2-¹⁸F-fluoro-2-deoxy-D-glucose (¹⁸F-FDG) which comprises treatment of a solid support-bound precursor of formula (Ib): 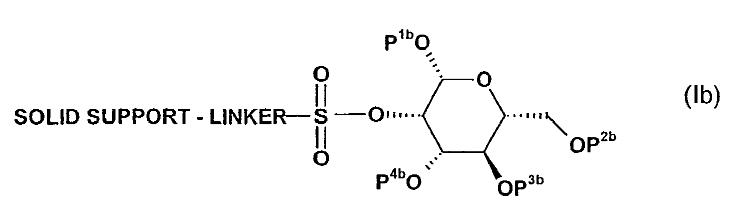 wherein P^{1b}, P^{2b}, P^{3b}, and P^{4b} are each independently hydrogen or a protecting group;
with ¹⁸F⁻ to produce the labelled tracer of formula (IIb) 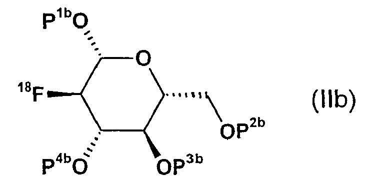 wherein P^{1b}, P^{2b}, P^{3b}, and P^{4b} are each independently hydrogen or a protecting group;
optionally followed by
(i) removal of excess ¹⁸F⁻, for example by ion-exchange chromatography; and/or
(ii) removal of the protecting groups; and/or
(iii) removal of organic solvent; and/or
(iv) formulation of the resultant compound of formula (IIb) as an aqueous solution.

In the compound of formula (Ib) the LINKER is preferably 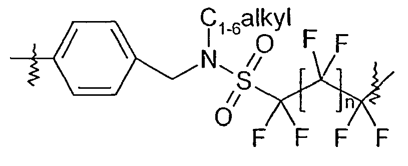 or 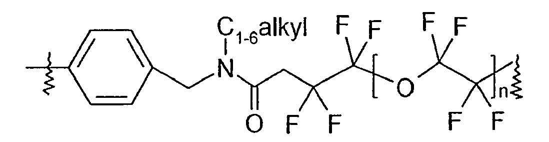 wherein n is 0 to 3, and is more preferably 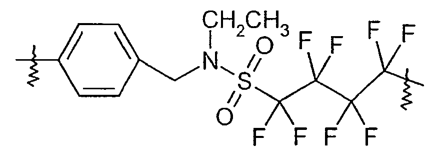 or 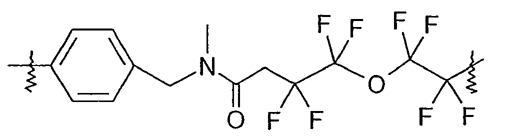 and the SOLID SUPPORT is suitably a polystyrene resin.

Removal of any protecting groups from the compound of formula (IIb) may be effected by standard methods as referred to above. In a preferred embodiment of this aspect of the invention, the sugar hydroxyl groups are protected as esters, suitably C₁₋₆ alkanoic esters, preferably as acetate esters, or as ethers, preferably C₁₋₆alkoxy methyl ethers, or acetals. Ester, acetal, or ether protecting groups may be conveniently removed by hydrolysis, for example in the presence of acid or base. Such deprotection may be effected on using solid supported acid or base catalysts that render the need for post deprotection neutralisation unnecessary

The present invention provides in a further aspect, a process for the production of 3'-deoxy-3'-¹⁸F-fluorothymidine (¹⁸F-FLT) which comprises treatment of a solid support-bound precursor of formula (Ic): 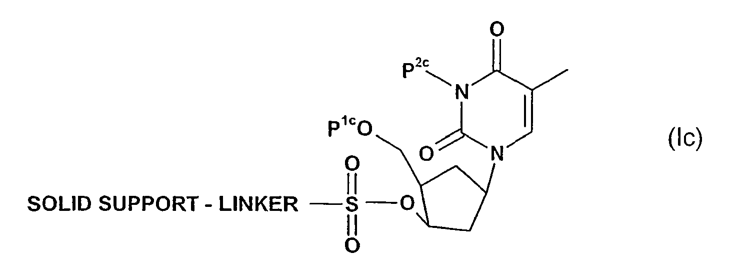 wherein P^{1c} and P^{2c} are each independently hydrogen or a protecting group;
with ¹⁸F⁻ to produce the labelled tracer of formula (IIc) 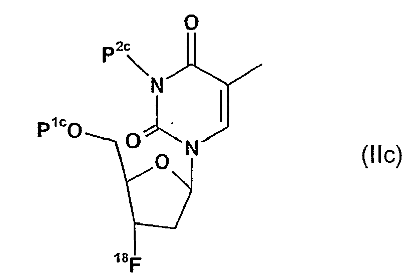 wherein P^{1c} and P^{2c} are each independently hydrogen or a protecting group;
optionally followed by
(i) removal of excess ¹⁸F⁻, for example by ion-exchange chromatography; and/or
(ii) removal of the protecting groups; and/or
(ii) removal of organic solvent; and/or
(iii) formulation of the resultant compound of formula (IIc) as an aqueous solution.

In this aspect of the invention, the amine and hydroxyl functional groups in the thymidine precursor are suitably protected using standard methods as referred to above. Suitably, the amine and hydroxyl groups are protected as esters, suitably C₁₋₆ alkyl esters, preferably as acyl esters. Ester protecting groups may be conveniently removed by hydrolysis, for example in the presence of acid or base. Such deprotection may be effected using a solid supported acid or base catalyst that renders the need for post deprotection neutralisation unnecessary

In the compound of (Ic), the Linker is preferably: 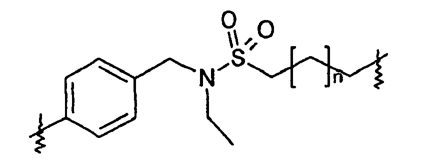 wherein n is 0 to 3.

In a further aspect of the invention, the TRACER precursor in the compound of formula (Ia) may be a peptide or protein such as a peptide comprising from 2 to 1,000 amino acids.

In a further aspect of the invention, there is provided a process for the production of 6-L-¹⁸F-fluorodopa (¹⁸F-FDOPA) which comprises treatment of a solid support-bound precursor of formula (Ig): 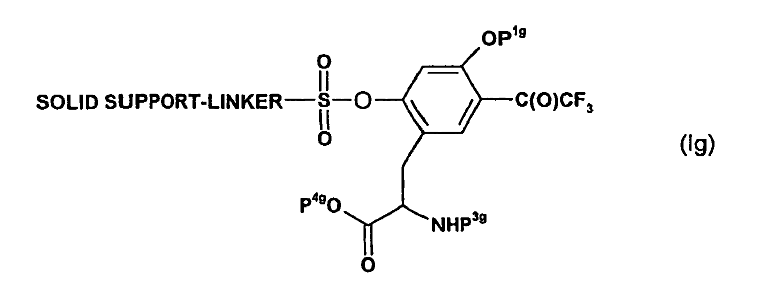 wherein P^{1g}, P^{3g}, and P^{4g} are each independently hydrogen or a protecting group such as t-butoxycarbonyl;
with ¹⁸F⁻ to produce the labelled tracer of formula (IIg) 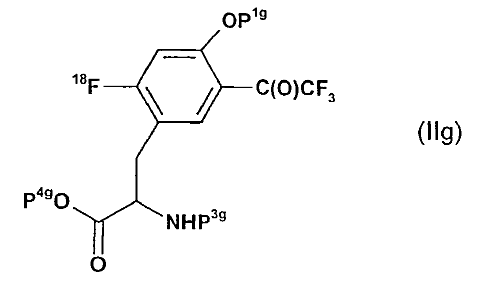 wherein P^{1g}, P^{3g}, and P^{4g} are each independently hydrogen or a protecting group such as t-butoxycarbonyl;
optionally followed by
(i) removal of excess ¹⁸F⁻ , for example by ion-exchange chromatography; and/or
(ii) conversion of the -C(O)CF₃ group to a hydroxyl group; and/or
(iii) removal of any protecting groups; and/or
(iv) removal of organic solvent; and/or
(v) formulation of the resultant FDOPA as an aqueous solution.

In this aspect of the invention, the hydroxyl functionality of the DOPA starting material are conveniently protected as esters, suitably C₁₋₆ alkanoic esters, preferably as acetate esters, or carbonate esters such as t-butoxycarbonyl esters. The acid functionality may be protected as a C₁₋₆ alkyl ester, preferably ethyl ester and the amine functionality may be protected as an amide preferably formyl or as a urethane, preferably as t-butoxycarbonyl urethane. Ester formyl and urethane protecting groups may be conveniently removed by hydrolysis, for example in the presence of acid or base. Such deprotection may be effected using a solid supported acid or base catalysts that render the need for post deprotection neutralisation unnecessary. Conversion of the -C(O)CF₃ group to a hydroxyl group, may be effected by treatment with an oxidising agent such as meta-chloro perbenzoic acid, followed by mild acidic hydrolysis. In this aspect of the invention, a particularly suitable LINKER is 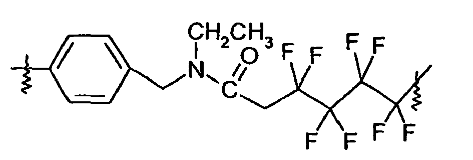 and the solid support is suitably a polystyrene resin.

The present invention provides in a further aspect, a process for the production of 2-(1,1-dicyanopropen-2-yl)-6-(2-fluoroethyl)-methytamino)-naphthalene (FDDNP) which comprises treatment of a solid support bound precursor of formula (Ih): 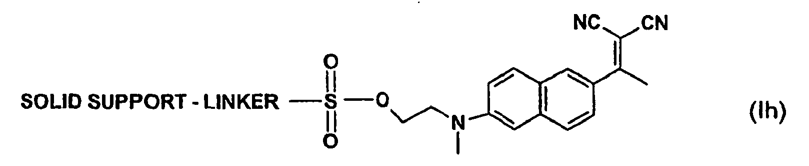 with ¹⁸F⁻ to produce the labelled tracer of formula (IIh) 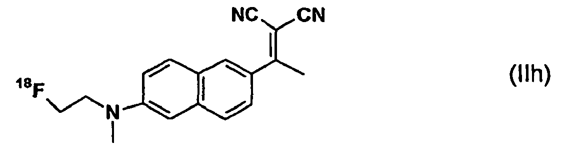 optionally followed by
(i) removal of unreacted ¹⁸F⁻ for example by ion-exchange chromatography; and/or
(ii) removal of organic solvent; and/or
(iii) formulation of the resultant compound of formula (IIh) as an aqueous solution.

In a further aspect, the invention provides a process for the production of an ¹⁸F-labelled tracer which comprises treatment of a solid support-bound precursor of formula (Id) 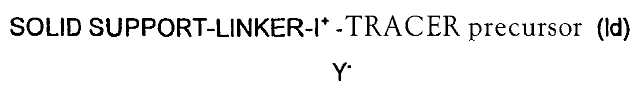 with ¹⁸F⁻ to produce the labelled tracer of formula (IId)
¹⁸F-TRACER (IId)
followed by optionally
(i) removal of excess ¹⁸F⁻, for example by ion-exchange chromatography; and/or
(ii) removal of any protecting groups; and/or
(iii) removal of organic solvent; and/or
(iv) formulation of the resultant compound of formula (IId) as an aqueous solution.

In the compound of formula (Id), the tracer is suitably an aryl containing compound such as a phenyl containing compound, preferably a substituted phenyl ring. In one such preferred aspect, the tracer prepared is FDOPA.

The compound of formula (1d) may be conveniently prepared from a functionalised commercially available resin such as a Merrifield Resin or Wang Resin. Suitably, a hydroxyiodoaryl (such as an iodophenol) containing LINKER group is treated with an inorganic base, such as cesium carbonate and then added to the resin, pre-swollen with an inert solvent, such as N,N-dimethylformamide and allowed to react at elevated temperature, for example 30 to 80°C. Excess reagents may be removed by washing the resin with further inert solvent. The resultant iodophenol functionalised resin may then be treated with a source of acetate anions (such as actetic acid, acetic anhydride, or acetyl chloride) in the presence of an oxidising agent, such as hydrogen peroxide to provide the corresponding diacetoxy-iodophenyl functionalised resin. The diacetoxy-iodophenyl functionalised resin may then be stirred in an inert solvent, such as dichloromethane, in the presence of acid such as hydrochloric acid, trifluoromethane sulphonic acid, or acetic acid at a low temperature, suitably -40°C to 10°C before addition of the tracer, suitably functionalised as a boronic acid or trialkyl tin derivative which may be coupled to the resin at a non-extreme temperature. As in previous steps, the desired compound of formula (Id) may be separated by filtration and washing with an inert solvent.

In the compound of formula (Id), the LINKER is as defined above but comprises an aryl group (suitably phenyl) adjacent to the I⁺. Preferred examples include

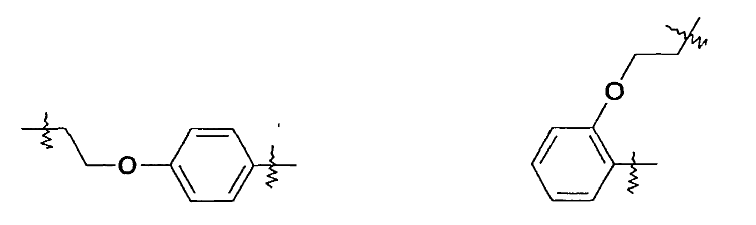

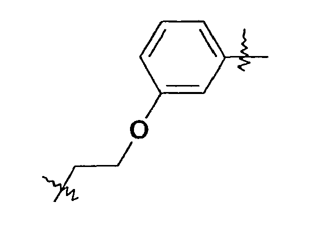

In the compound of formula (Id), Y⁻ is an anion, preferably trifluoromethylsulphonate (triflate) anion.

The present invention provides in a further aspect, a process for the production of 6-L-¹⁸F-fluorodopa (¹⁸F-FDOPA) which comprises treatment of a solid support-bound precursor of formula (Ie): 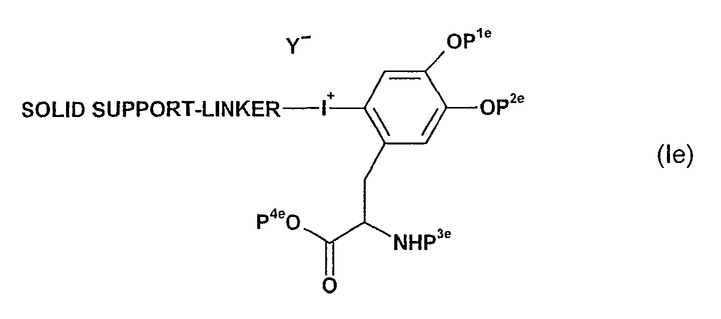 wherein P^{1e}, P^{2e}, P^{3e}, and P^{4e} are each independently hydrogen or a protecting group and Y⁻ is an anion such as triflate;
with ¹⁸F⁻ to produce the labelled tracer of formula (IIe) 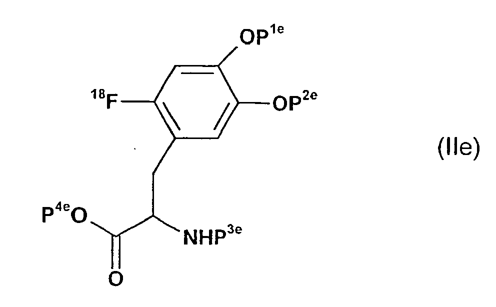 wherein P^{1e}, P^{2e}, P^{3e}, and P^{4e} are each independently hydrogen or a protecting group;
optionally followed by
(i) removal of excess ¹⁸F⁻, for example by ion-exchange chromatography; and/or
(ii) removal of any protecting groups; and/or
(iii) removal of organic solvent; and/or
(iv) formulation of the resultant compound of formula (IIe) as an aqueous solution.

In this aspect of the invention, the hydroxyl, amine, and acid functionality of the DOPA starting material are conveniently protected as esters, suitably C₁₋₆ alkyl esters, preferably as acyl esters such as t-butoxycarbonyl, or ethers, preferably as C₁₋₆ alkyl ethers, or amides. These protecting groups may be conveniently removed by hydrolysis, for example in the presence of acid or base. Such deprotection may be effected using a solid supported acid or base catalysts that render the need for post deprotection neutralisation unnecessary.

In the compounds of formula (Ie), preferred LINKER groups are as described for the compounds of formula (Id) and the SOLID SUPPORT is suitably a polystyrene resin.

The present invention provides in a further aspect, a process for the production of 2-, 5- or 6-fluoro-3-(2(s)-azetidinylmethoxy)pyridines which comprises treatment of a solid support-bound precursor of formula (If): 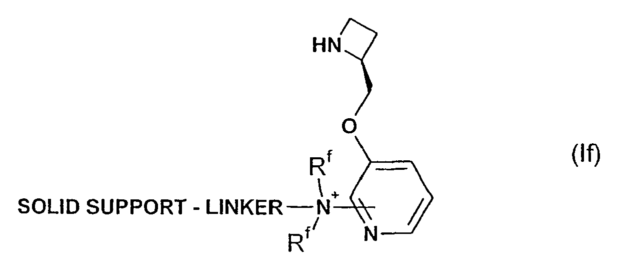 wherein the groups R^{f} are each independently selected from C₁₋₆ alkyl; with ¹⁸F⁻ to produce the labelled tracer of formula (IIf) 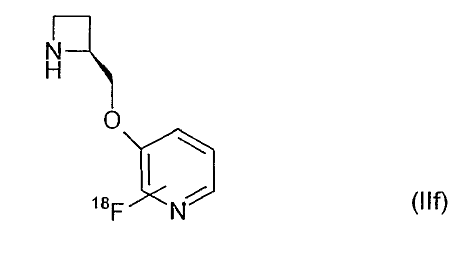 optionally followed by
(i) removal of excess ¹⁸F⁻, for example by ion-exchange chromatography; and/or
(ii) removal of organic solvent; and/or
(iii) formulation of the resultant compound of formula (IIf) as an aqueous solution.

Some of the compounds of formula (I) are novel and thus form a further aspect of the present invention. Thus, for example, compounds of formula (Ia), in particular those of formula (Ib), (Ic), (Ig) and (Ih), and compounds of formula (Id), in particular those of formula (Ie) all as defined above, form separate aspects of the present invention.

As described above, the advantages of such solid-phase processes for preparation of ¹⁸F-labelled tracers include the relative speed of the process, simplified purification methods and ease of automation- all of which mean that the processes are suitable for preparation of ¹⁸F-labelled tracers for use in PET. Accordingly, the present invention provides the use of a process for the manufacture of a ¹⁸F-labelled tracer of formula (II) or (IIb to IIh) for use in PET.

Conveniently, the solid support bound precursor of formula (I) could be provided as part of a kit to a radiopharmacy. The kit may contain a cartridge which can be plugged into a suitably adapted automated synthesiser. The cartridge may contain, apart from the solid support-bound precursor, a column to remove unwanted fluoride ion, and an appropriate vessel connected so as to allow the reaction mixture to be evaporated and allow the product to be formulated as required. The reagents and solvents and other consumables required for the synthesis may also be included together with a compact disc carrying the software which allows the synthesiser to be operated in a way so as to meet the customers requirements for radioactive concentration, volumes, time of delivery etc.

Conveniently, all components of the kit are disposable to minimise the possibilities of contamination between runs and may be sterile and quality assured.

The invention further provides a radiopharmaceutical kit for the preparation of an ¹⁸F-labelled tracer for use in PET, which comprises:
(i) a vessel containing a compound of formula (I) or (Ia to Ih); and
(ii) means for eluting the vessel with a source of ¹⁸F⁻;
(iii) an ion-exchange cartridge for removal of excess ¹⁸F⁻; and optionally
(iv) a cartridge for solid-phase deprotection of the resultant product of formula (II) or (IIb to IIh).

The invention further provides a cartridge for a radiopharmaceutical kit for the preparation of an ¹⁸F-labelled tracer for use in PET which comprises:
(i) a vessel containing a compound of formula (I) or (Ia to Ih); and
(ii) means for eluting the vessel with a source of ¹⁸F⁻.

The invention will now be illustrated by way of the following Examples.
Throughout the Examples, abbreviations used are as follows:
DMF: N,N-dimethylformamide
w/v: weight/volume
h : hour(s)
tlc : thin layer chromatography
THF : tetrahydrofuran
eq. : equivalents

### Examples

### Example 1. Synthesis of 2[¹⁸F]-fluoro-2-deoxy-D-glucose (FDG)

### Intermediate 1

### Preparation of Methyl -4,6-O- benzylidine-3-ethoxy methyl α-D-mannopyranoside

### Step 1: Synthesis of Methyl 4,6-O- benzylidine-α-D-glucopyranoside

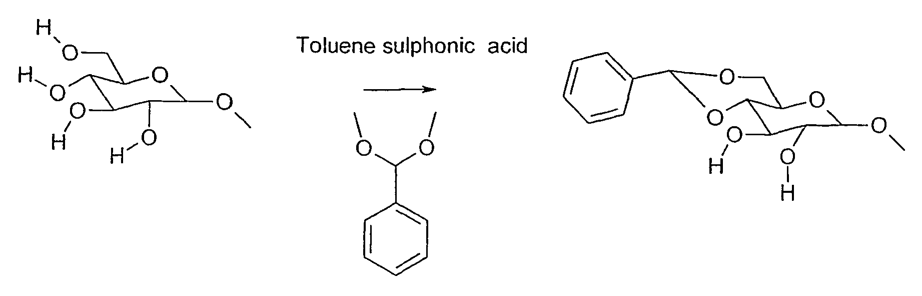

Following literature Evans, M. E. Carbohydrate Research (1972), 21 (3), 473-5, Methyl- α -D-glucopyranoside (Aldrich, 257mmol) in DMF (200ml) was treated with α,α-dimethoxy toluene 39.0 g 257mmol) and toluene sulphonic acid monohydrate 100mg in a 1l round bottomed flask. This was attached to a Buchi and evacuated and rotated. The flask was lowered into a water bath at 65°C and the DMF allowed to gently reflux into the vapour duct but not to distil out. The temperature of the water bath was then raised to 100 °C and the DMF distilled from the reaction. When the distillation of the reaction was complete the reaction was cooled and treated with a solution of sodium hydrogen carbonate (5g) in water (750ml) and ethyl alcohol (250ml). The reaction was heated to 95°C on a water bath and stirred until the product became finely dispersed. The reaction was then cooled to 4°C and the product filtered off washed well with water and dried in vacuum.
m.p. 207-208.5

### Step 2 Preparation of Methyl 4,6-O- benzylidine-3- ethoxymethyl-α-D-glucopyranoside

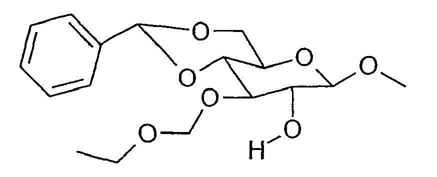

Methyl-4,6-O-benzylidine-α-D-glucopyranoside,(19.2g,68mmole), ethoxymethylchloride (9.7g, 81.6mmol) and tetrabutylammonium hydroxide (5ml of a 40% w/v solution) in dichloromethane (150ml) was stirred vigorously with a 10% aqueous solution of sodium hydroxide (200ml) at room temperature. After 5 hours the aqueous phase was replaced with a fresh solution of 10% aqueous sodium hydroxide (200ml) to which tetrabutylammonium hydroxide (5ml of a 40% w/v solution) was added, and rapid stirring continued overnight. The organic phase was then separated dried over sodium sulphate and evaporated in vacuum. Thin layer chromatography of the residue (40-60 hexanes-ethyl acetate 2:1) on silica developed by spraying with ceric ammonium molybdate (see above) indicated the presence of three new alkylated products. Chromatography on silica (1kg, dry weight) in a gradient of 40-60 hexanes-ethyl acetate 2:1 to 1:1 gave three fractions which NMR indicated to be
Fraction 1: Methyl-2,3, - diethoxymethyl 4,6-O- benzylidine-α-D-glucopyranoside
Fraction 2: Methyl-2- ethoxymethyl 4,6-O- benzylidine-α-D-glucopyranoside
Fraction 3: Methyl 3- ethoxymethyl 4,6-O- benzylidine-α-D-glucopyranoside

### Step 3 Preparation of Methyl 2 keto 3-ethoxymethyl 4,6-O- benzylidine-α-D-glucopyranoside

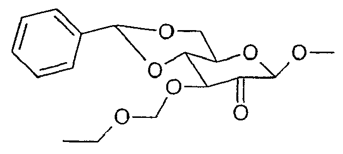

Methyl 4,6-O- benzylidine-3- ethoxymethyl α -D-glucopyranoside (3g, 8.0mmol) was treated with methylsulfoxide (50ml) and acetic anhydride (25ml) at room temperature for 24 h until the reaction will be adjudged completed by tlc (Petrol ether/ Ethyl acetate 1:1) developed with cerium ammonium molybdate. The solution was then diluted with diethyl ether (200ml) and washed with 10% aqueous potassium carbonate solution to hydrolyse the excess acetic anhydride. The ether layer was separated and washed with water (100ml). The ether layer was separated, dried over sodium sulphate and concentrated in vacuum to give a crystalline solid. Recrystallization from ether/petrol gave 1.5 g of Methyl 2 keto-3-ethoxymethyl 4,6-O- benzylidine- α -D-glucopyranoside.

### Step 4 Preparation of Methyl 4,6-O- benzylidine-3-ethoxymethyl α-D-mannopyranoside

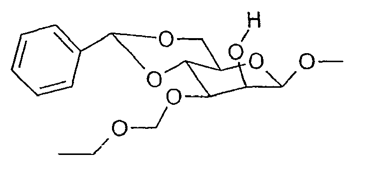

Methyl 2 keto 3-ethoxymethyl 4,6-O-benzylidine-α-D-glucopyranoside (0.5g 1.3mmol) in methanol (50ml) THF (10ml) was treated with sodium borohydride (200mg, 5.3mmol) at room temperature with continuous stirring. The reaction was then concentrated in vacuum to a gum and the product partitioned between ethyl acetate (50ml) and 10% aqueous potassium carbonate solution (50 ml). The ethyl acetate solution was separated, dried over sodium sulphate and concentrated in vacuo to give methyl 3-ethoxymethyl 4,6-O- benzylidine- α -D-mannopyranoside.

### Example 1(i) Preparation of perfluorobutane-1,4-bis-sulphonylchloride

(Following the method of Weiming Qiu and Donald J. Burton Journal of fluorine chemistry, 60 (1993) 93-100.)

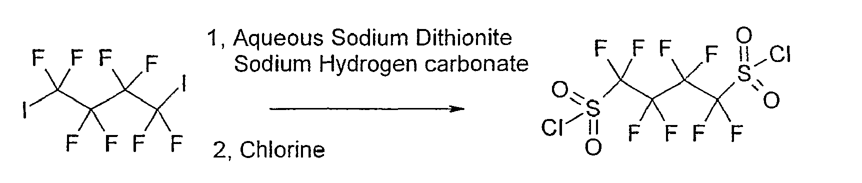

The mixture of 1,4 diiodoperfluorobutane (I(CF₂)₄I) (24.14g, 53.2mmol), sodium dithionite Na₂S₂O₄ (24g, 117.2mmol) and sodium hydrogen sulphate NaHCO₃ (12.8g, 152.4mmol) in water H₂O (36ml) / Acetonitrile CH₃CN (36ml) was stirred at room temperature for 2 hours. It was filtered, and the filtrate was concentrated under reduced pressure to remove the acetonitrile. To the residue was added H₂O (100ml). The so obtained solution was vigorously stirred and treated with chlorine gas Cl₂ at 0°C until the colour of I₂ disappeared. Dichloromethane CH₂Cl₂ (100ml) was added and the mixture vigorously shaken. The organic phase was separated, and the aqueous phase was extracted with CH₂Cl₂. The combined organic phase was washed with water H₂O, brine, and dried with sodium sulphate Na₂SO₄ and concentrated to afford a waxy yellow crystalline solid. (15.4g, 74%). Recrystallization from hexane afforded off-white needles of pertluorobutane-1,4-bis-sulphonylchloride.
¹⁹F NMR (CDCl₃, CFCl₃ reference) δ: -104.4, -119.1.

### Example 1 (ii) Preparation of perfluorobutane-1,4-bis-suphonate dipotassium salt

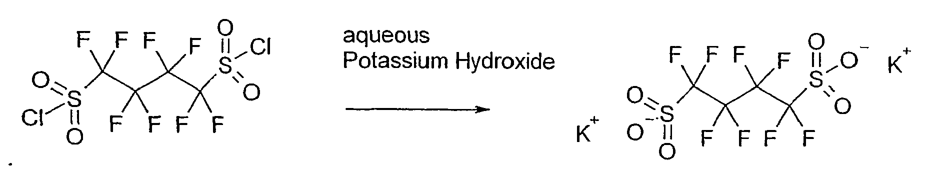

To the solution of potassium hydroxide KOH (9.8g, 5eq) in water H₂O (19ml) was added gradually pertluorobutane-1,4-bis-sufphonylchloride (14g, 35mmol) at 85°C-90°C with stirring. After the addition, the reaction was continued for more 4 hours at the same temperature, and then it was cooled overnight. It was filtered and the solids was washed with a little of cooled water and dried in vacuum to give perfluorobutane-1,4-bis-sulphonate dipotassium salt
¹⁹F NMR (CD₃OD, CFCl₃ reference) δ : -114.00, -120.11.

### Example 1(iii) Preparation of perfluorobutane-1,4-bis-sulphonic acid

(Following the method described in US patent 4329,478, Fred E. Behr.)

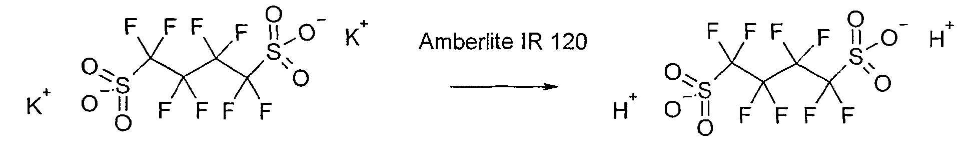

Perfluorobutane-1,4-bis-sulphonate dipotassium salt (15g, 34.2mmol) was dissolved in hot water (100ml). It was added to an ion exchange column of Amberlyst 15 resin, (40x4cm) which had been previously washed with excess 6N HCl and rinsed with distilled water. The column was then washed slowly with distilled water, and the first 300ml of aqueous solution collected. The solution was concentrated in vacuum and the residue was dried under reduced pressure at 80°C to afford perfluorobutane-1,4-bis-sulphonic acid. (11.0g, 30mmol, 88%)
¹H NMR (CDCl₃,) δ : 8.00
¹⁸F NMR (CDCl₃, CFCl₃ reference) δ : -114.7, -121.3.

### Example 1 (iv) Preparation of perfluorobutane-1,4-bis-sulphonic acid anhydride (Following the method described in US patent 4329,478, Fred E. Behr.)

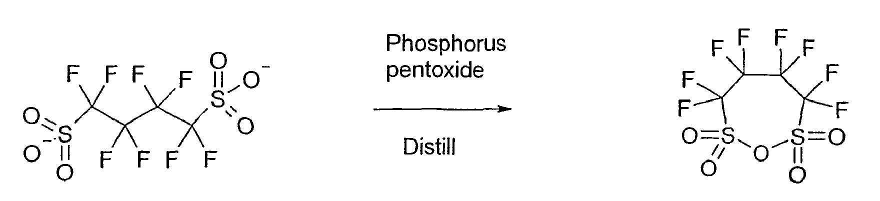

Perfluorobutane-1,4-bis-sulphonic acid (11.0g, ~30mmol) was mixed with P₂O₅ (40g, ~10eq) and sand. The mixture was heated to 140-180°C and distilled under reduced pressure with dry-ice cooling collector to afford crude product (5.12g). Redistilation gives pure perfluorobutane-1,4-bis-sulphonic acid anhydride.
¹⁸F NMR (CDCl₃, CFCl₃ reference) δ: -105.7, -121.8.

### Example 1(v) Synthesis of PS - 4-(Benzyl-ethyl-sulfonamide)octafluoro-butane-1- sulfonic acid

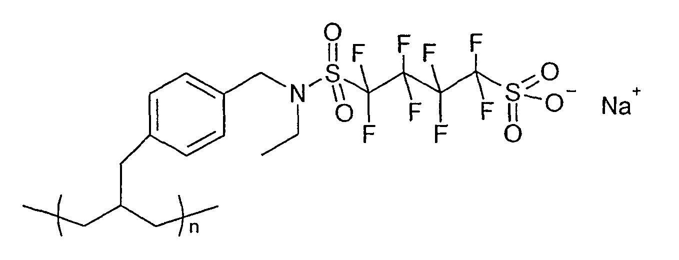

To a portion of the polystyrene resin (Novabiochem, Novasyn resin) (202mg), which had previously been swollen in dichloromethane (2ml) and then suspended in a further aliquot of dichloromethane (2ml) the perfluorobutyl-1,4-cyclic-sulfonic anyhydride (116mg, 5Eq) was added. Following this di-isopropyethyl amine (0.174 ml) was added and the suspension stirred overnight at room temperature. The solvent was removed by filtration and the resin washed with consecutive addition and filtration of dichloromethane (5 ml), methanol (5ml), DMF (5ml), water (5 ml), methanol (5 ml), and dichloromethane (5ml). The resulting resin was then treated with NaOH (1M) in THF/water (2 x 2ml) before washing with consecutive portions of methanol (5ml), dichloromethane (5ml) and methanol (5 ml) again. The resin was then dried under high vacuum.
Gel Phase ¹⁹F NMR (referenced to CFCl₃ ,300K) : δ-121.0, -114.8, -113.4

### Example 1 (vi) Synthesis of PS - 4-(Benzyl-ethyl-sulfonamide)octafluoro-butane-1-sulfonyl chloride

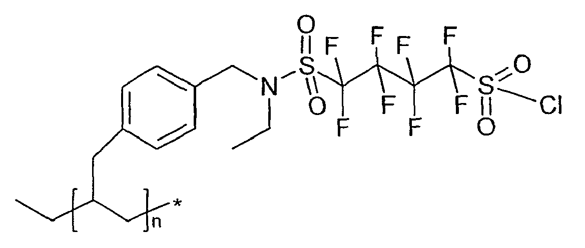

A portion of the resin prepared in the manner of Example 1(v) above is swollen with dichloromethane (2ml) and then washed consecutively with HCl (1M) in THF/water (10 x 5 ml) to give the free sulphonic acid. The resin is washed consecutively with dichloromethane, methanol and THF before drying under high vacuum.

The resin is then suspended in dichloromethane and to it is added in excess a common chlorinating agent such as phosphorous pentachloride, phosphorus trichloride or thionyl chloride. The suspension is stirred for 2 hours before filtration and then washing of the resin with dichloromethane and then THF.

### Example 1 (vii) Synthesis of protected mannopyranose resin

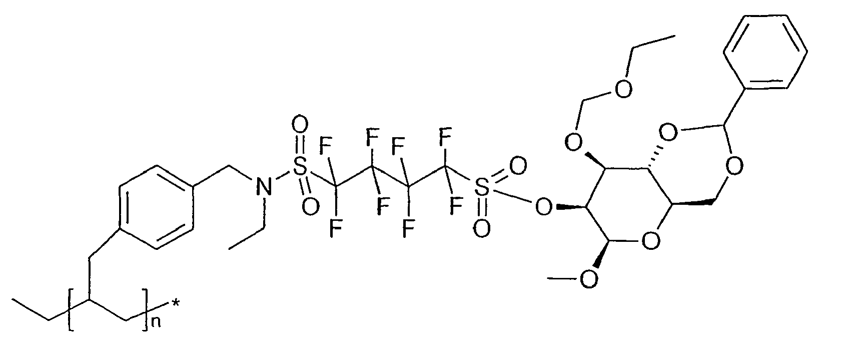

A solution of Intermediate 1 in THF was added to a portion of the resin prepared as described in Example 1 (vi) above which has previously been swollen in THF. To this is added a solution of potassium *t*-butoxide in tetrahydrofuran and the suspension is stirred overnight. After filtration the resin is washed consecutively with dichloromethane and THF before drying under high vacuum.

### Example 1(viii) Radiofluorination to prepare [¹⁸F]-FDG

To a portion of the resin (prepared as described in Example 1 (vii)) held in a cartridge is added a solution in dry acetonitrile of kryptofix, potassium carbonate and [¹⁸F]-fluoride. The suspension is heated to 85°C for 10 minutes and then the solution is filtered off. The solution is then passed onto a C₁₈ solid phase extraction cartridge and washed with water to remove acetonitrile, kryptofix and potassium carbonate. Addition of more acetonitrile washes the radiofluorinated product of the cartridge into a solution of 0.1 M HCl. This solution is heated for 5 minutes before neutralization and analysis.

### Example 2 Synthesis of 2-(1,1-dicyanopropen-2-yl)-6-(2-[¹⁸F]-fluoroethyl)-(methylamino)-napthalene (FDDNP)

### Example 2(i) - Synthesis of PS - 4-(Benzyl-ethyl-sulfonamide)-butane-1-sulfonyl chloride

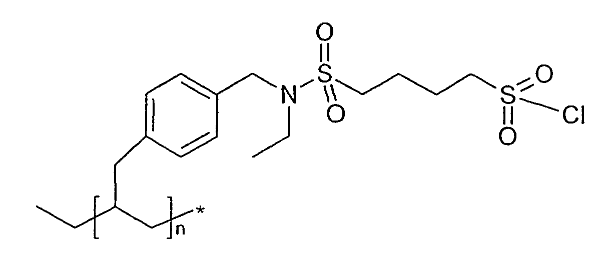

To a suspension of the resin that has been swollen in dichloromethane (5ml) excess 1,4-butane-disulfonyl chloride in dichloromethane is added together with an excess of triethylamine. The suspension is stirred at room temperature overnight. After filtration the resin is washed consecutively with dichloromethane, methanol, THF, water, methanol and another portion of dichloromethane. After the final washing the resin is dried under vacuum.

### Example 2(ii) - Synthesis of 2-(1,1-dicyanopropen-2-yl)-6-(2-ethyl)-(methylamino)-napthalene resin

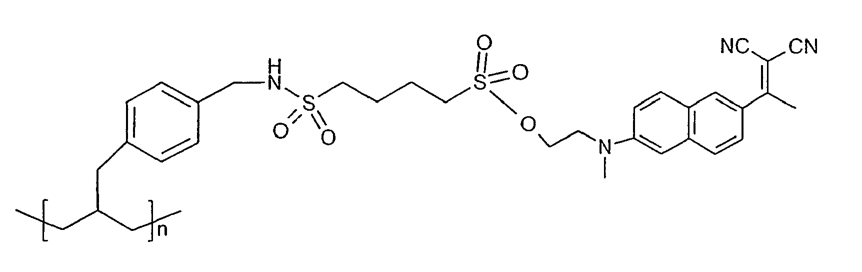

To a suspension of the resin above that has been swollen in dichloromethane (2 ml), excess 2-(1,1-dicyanopropen-2-yl)-6-(2-hydroxyethyl)-methylamino)-naphthalene in dichloromethane is added together with an excess of triethylamine. The suspension is stirred at room temperature overnight. After filtration the resin is washed consecutively with dichloromethane and THF. After the final washing the resin is dried under vacuum.

### Example 2(iii)- Radiofluorination to prepare [¹⁸F]-FDDNP

To a portion of the resin held in a cartridge is added a solution in dry acetonitrile of kryptofix, potassium carbonate and [¹⁸F]-fluoride. The suspension is heated to 85°C for 10 minutes and then the solution is filtered off. The resin is washed with acetonitrile (1ml) and all the contents collected together before evaporation of the solvent prior to formulation.

### Example 3 - Synthesis of [¹⁸F]-fluorobenzene

### Example 3 (i) Synthesis of PS iodo-phenyl benzyl ether

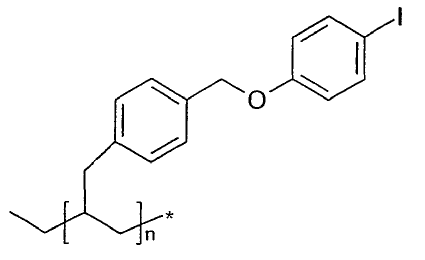

To a suspension of Wang Resin pre-swollen in DMF (2ml) a solution of cesium carbonate and iodophenol in DMF were added. The mixture was stirred for 3h at 60°C and then left at room temperature overnight. After filtration the resin was washed consecutively with methanol, dichloromethane, DMF and THF before thorough drying under high vacuum.

### Example 3 (ii) Synthesis of PS diacetoxy-iodo-phenyl benzyl ether

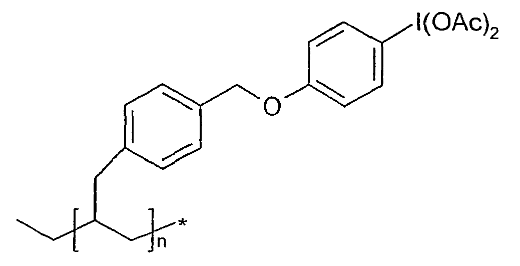

A suspension of the resin above was treated with acetic anhydride and hydrogen peroxide (see method of S.Ficht, Tetrahedron, 57 (2001) 4863) in a 4:1 ratio at 40°C overnight. The resin was then filtered and washed thoroughly with methanol and then dried under high vacuum until dry.

### Example 3 (iii) - Synthesis of PS (phenyl)(4-phenyl benzyl ether)iodonium triflate

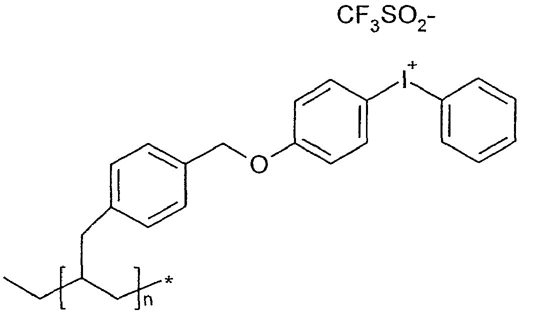

To a suspension of the resin from above in dichloromethane, trifluoromethane sulfonic acid is added dropwise at a temperature of -30°C for 15 minutes. The mixture is then warmed to 0°C for a further 15 minutes before being stirred at room temperature overnight. The suspension is then cooled to -30°C and phenyl boronic acid is added, and the suspension is stirred for 1h before warming to room temperature and further stirring overnight The mixture is then filtered and washed thoroughly with dichloromethane and diethyl ether before drying under vacuum.

### Example 3(iv) - Radiofluorination to prepare [¹⁸F] fluorobenzene

To a portion of the resin held in a cartridge is added a solution in dry acetonitrile of kryptofix, potassium carbonate and [¹⁸F]-fluoride. The suspension is heated to 85°C for 10 minutes and then the solution is filtered off. The resin is washed with acetonitrile (1ml) and all the contents collected together before evaporation of the solvent prior to formulation.

## Claims

1. A process for the production of an ¹⁸F-labelled tracer which comprises treatment of a resin-bound precursor of formula (I)
SOLID SUPPORT-LINKER-X-TRACER precursor (I)
wherein X is a group which promotes nucleophilic substitution at a specific site on the attached TRACER precursor;
with ¹⁸F⁻ to produce the labelled tracer of formula (II)
¹⁸F-TRACER (II).

2. A process for the production of an ¹⁸F-labelled tracer according to claim 1 which comprises treatment of a resin-bound precursor of formula (Ia)
SOLID SUPPORT-LINKER-SO₂-O -TRACER precursor (Ia)
with ¹⁸F⁻ to produce the labelled tracer of formula (II)
¹⁸F-TRACER (II)
followed by optionally
(i) removal of excess ¹⁸F⁻ , for example by ion-exchange chromatography; and/or
(ii) removal of any protecting groups; and/or
(iii) removal of organic solvent; and/or
(iv) formulation of the resultant compound of formula (II) as an aqueous solution.

3. A process according to claim 1 or 2 for the production of 2-¹⁸F-fluoro-2-deoxy-D-glucose (¹⁸F-FDG) which comprises treatment of a solid support-bound precursor of formula (Ib): 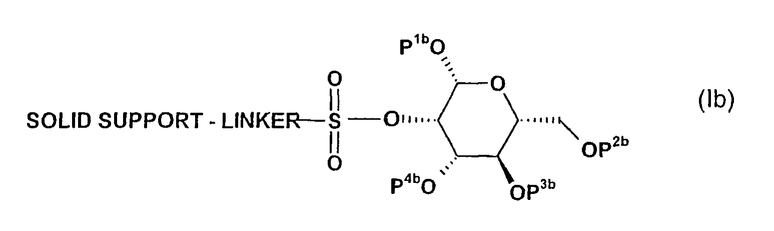 wherein P^{1b}, P^{2b}, P^{3b}, and P^{4b} are each independently hydrogen or a protecting group;
with ¹⁸F⁻ to produce the labelled tracer of formula (IIb) 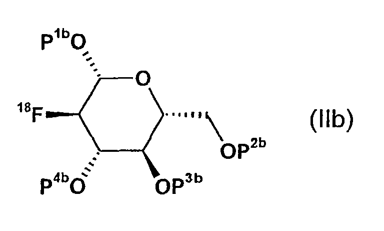 wherein P^{1b}, P^{2b}, P^{3b}, and P^{4b} are each independently hydrogen or a protecting group;
optionally followed by
(i) removal of excess ¹⁸F⁻ , for example by ion-exchange chromatography; and/or
(ii) removal of the protecting groups; and/or
(iii) removal of organic solvent; and/or
(iv) formulation of the resultant compound of formula (IIb) as an aqueous solution.

4. A process according to claim 1 or 2 for the production of 3'-deoxy-3'-¹⁸F-fluorothymidine (¹⁸F-FLT) which comprises treatment of a solid support-bound precursor of formula (Ic) : 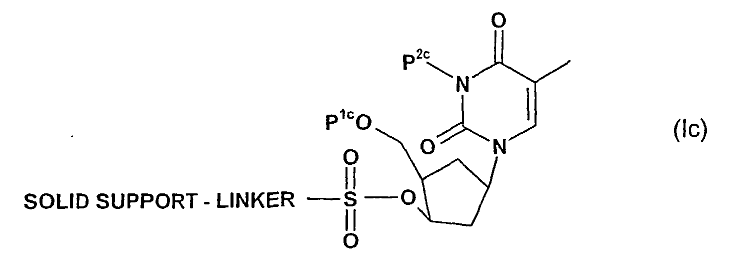 wherein P^{1c} and P^{2c} are each independently hydrogen or a protecting group;
with ¹⁸F⁻ to produce the labelled tracer of formula (IIc) 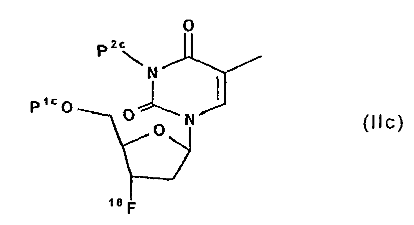 wherein P^{1c} and P^{2c} are each independently hydrogen or a protecting group;
optionally followed by
(i) removal of excess ¹⁸F⁻ , for example by ion-exchange chromatography; and/or
(ii) removal of the protecting groups; and/or
(iii) removal of organic solvent; and/or
(iv) formulation of the resultant compound of formula (IIc) as an aqueous solution.

5. A process according to claim 1 or 2 for the production of 2-(1,1-dicyanopropen-2-yl)-6-(2-fluoroethyl)-methylamino)-naphthalene (FDDNP) which comprises treatment of a solid support bound precursor of formula (1h): 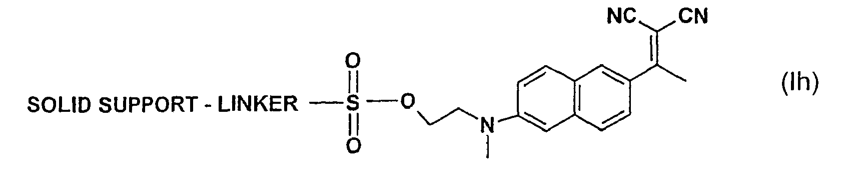 with ¹⁸F⁻ to produce the labelled tracer of formula (IIh) 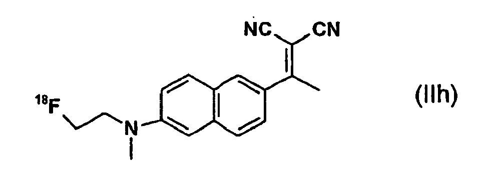 optionally followed by
(i) removal of unreacted ¹⁸F⁻, for example by ion-exchange chromatography; and/or
(ii) removal of organic solvent; and/or
(iii) formulation of the resultant compound of formula (IIh) as an aqueous solution.

6. A process according to claim 1 for the production of an ¹⁸F-labelled tracer which comprises treatment of a solid support-bound precursor of formula (Id) 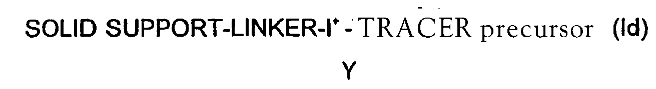 Y⁻ is an anion, preferably trifluoromethylsulphonate (triflate) anion.
with ¹⁸F⁻ to produce the labelled tracer of formula (IId)
¹⁸F-TRACER (IId)
followed by optionally
(i) removal of excess ¹⁸F⁻, for example by ion-exchange chromatography; and/or
(ii) removal of any protecting groups; and/or
(iii) removal of organic solvent; and/or
(iv) formulation of the resultant compound of formula (IId) as an aqueous solution.

7. A process according to claim 1 or 6 for the production of 6-L-¹⁸F-fluorodopa (¹⁸F-FDOPA) which comprises treatment of a solid support-bound precursor of formula (Ie): 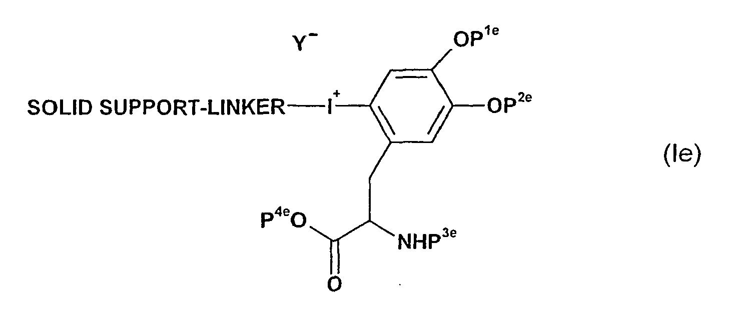 wherein P^{1e}, P^{2e}, P^{3e}, and P^{4e} are each independently hydrogen or a protecting group and Y⁻ is an anion, preferably trifluoromethylsulphonate (triflate) anion.; with ¹⁸F to produce the labelled tracer of formula (IIe) 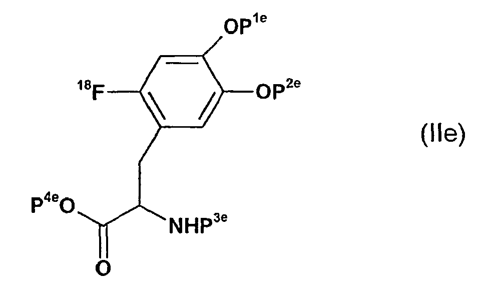 wherein P^{1e}, P^{2e}, P^{3e}, and P^{4e} are each independently hydrogen or a protecting group;
optionally followed by
(i) removal of excess ¹⁸F⁻ for example by ion-exchange chromatography; and/or
(ii) removal of any protecting groups; and/or
(iii) removal of organic solvent; and/or
(iv) formulation of the resultant compound of formula (IIe) as an aqueous solution.

8. A process for the manufacture of a ¹⁸F-labelled tracer of formula (II), according to any one of claims 1 to 7, for use in PET.

9. A compound of formula (Ib) 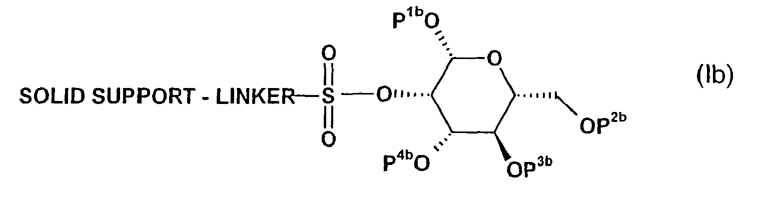 wherein P^{1b}, P^{2b}, P^{3b}, and P^{4b} are each independently hydrogen or a protecting group.

10. A compound of formula (Ic): 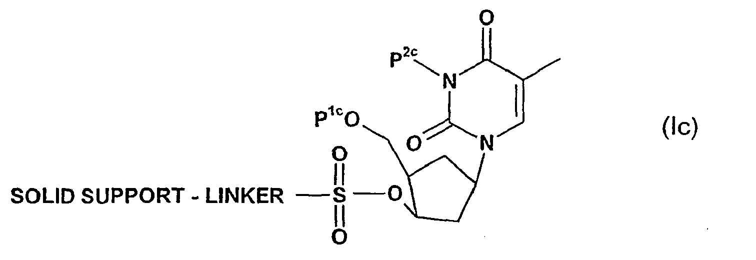 wherein P^{1c} and P^{2c} are each independently hydrogen or a protecting group.

11. A compound of formula (Ih):

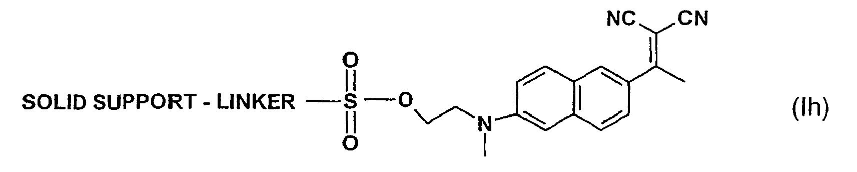

12. A compound of formula (Ie): 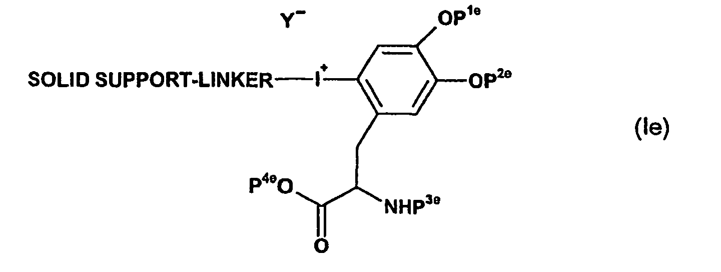 wherein P^{1e}, P^{2e}, P^{3e}, and P^{4e} are each independently hydrogen or a protecting group and Y⁻ is an anion such as triflate.

13. A radiopharmaceutical kit for the preparation of an ¹⁸F-labelled tracer for use in PET, which comprises:
(i) a vessel containing a compound of formula (I) or (Ia), (Ib), (Ic), (Id), (le), or (Ih), as defined in any one of claims 1 to 7; and
(ii) means for eluting the vessel with a source of ¹⁸F⁻;
(iii) an ion-exchange cartridge for removal of excess ¹⁸F⁻; and optionally
(iv) a cartridge for solid-phase deprotection of the resultant product of formula (II) or (IIb), (IIc), (IId), (IIe), or (IIh), as defined in any one of claims 1 to 7.

14. A cartridge for a radiopharmaceutical kit for the preparation of an ¹⁸F-labelled tracer for use in PET which comprises:
(i) a vessel containing a compound of formula (I) or (Ia), (Ib), (Ic), (Id), (Ie), or (Ih), as defined in any one of claims 1 to 7; and
(ii) means for eluting the vessel with a source of ¹⁸F⁻.

## Patentansprüche

1. Verfahren zur Herstellung eines ¹⁸F-markierten Tracers, umfassend eine Behandlung eines an ein Harz gebundenen Vorläufers der Formel (I)
FESTER TRÄGER-LINKER-X-TRACER-VORLÄUFER (I)
wobei X eine Gruppe ist, die eine nucleophile Substitution an einer spezifischen Stelle auf dem gebundenen Tracer-Vorläufer fördert,
mit ¹⁸F⁻, um den markierten Tracer der Formel (II) herzustellen
¹⁸F-TRACER (II).

2. Verfahren zur Herstellung eines ¹⁸F-markierten Tracers nach Anspruch 1, umfassend eine Behandlung eines an ein Harz gebundenen Vorläufers der Formel (la)
FESTER TRÄGER-LINKER-SO₂-O-TRACER-VORLÄUFER (la)
mit ¹⁸F, um den markierten Tracer der Formel (II) herzustellen
¹⁸F-TRACER (II)
gefolgt von gegebenenfalls
(i) Entfernung von überschüssigem ¹⁸F⁻, z. B. durch lonenaustauschchromatographie; und/oder
(ii) Entfernung beliebiger Schutzgruppen; und/oder
(iii) Entfernung von organischem Lösungsmittel; und/oder
(iv) Formulierung der resultierenden Verbindung der Formel (II) als eine wässrige Lösung.

3. Verfahren nach Anspruch 1 oder 2 zur Herstellung von 2-¹⁸F-Fluoro-2-desoxy-D-glukose (¹⁸F-FDG), umfassend eine Behandlung eines an einen festen Träger gebundenen Vorläufers der Formel (Ib): 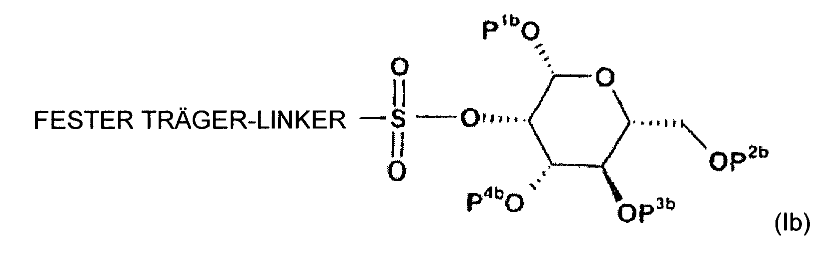 wobei P^{1b}, P^{2b}, P^{3b} und P^{4b} jeweils unabhängig für Wasserstoff oder eine Schutzgruppe stehen;
mit ¹⁸F⁻, um den markierten Tracer der Formel (IIb) herzustellen 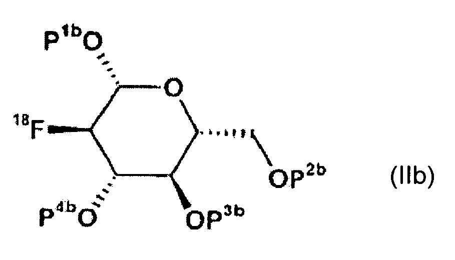 wobei P^{1b}, P^{2b}, P^{3b} und P^{4b} jeweils unabhängig für Wasserstoff oder eine Schutzgruppe stehen;
gegebenenfalls gefolgt von
(i) Entfernung von überschüssigem ¹⁸F⁻, z. B. durch lonenaustauschchromatographie; und/oder
(ii) Entfernung der Schutzgruppen; und/oder
(iii) Entfernung von organischem Lösungsmittel; und/oder
(iv) Formulierung der resultierenden Verbindung der Formel (IIb) als eine wässrige Lösung.

4. Verfahren nach Anspruch 1 oder 2 zur Herstellung von 3'-Desoxy-3'-¹⁸F-fluorothymidin (¹⁸F-FLT), umfassend eine Behandlung eines an einen festen Träger gebundenen Vorläufers der Formel (Ic): 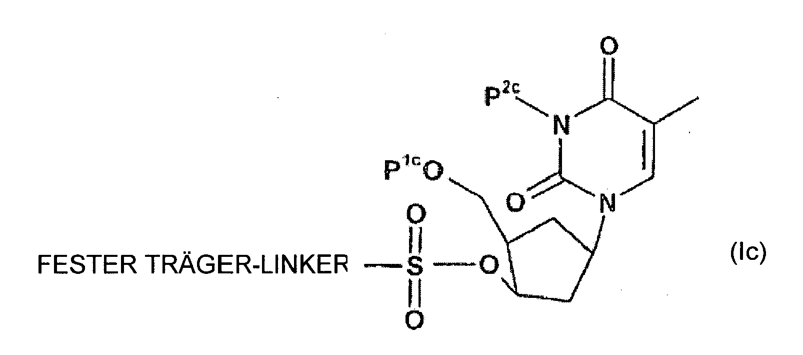 wobei P^{1c} und P^{2c} jeweils unabhängig für Wasserstoff oder eine Schutzgruppe stehen;
mit ¹⁸F⁻, um den markierten Tracer der Formel (IIc) herzustellen 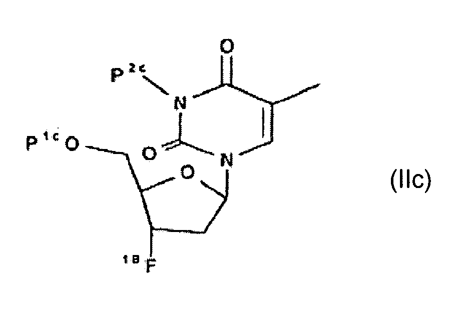 wobei P^{1c} und P^{2c} jeweils unabhängig für Wasserstoff oder eine Schutzgruppe stehen;
gegebenenfalls gefolgt von
(i) Entfernung von überschüssigem ¹⁸F⁻, z. B. durch lonenaustauschchromatographie; und/oder
(ii) Entfernung der Schutzgruppen; und/oder
(iii) Entfernung von organischem Lösungsmittel; und/oder
(iv) Formulierung der resultierenden Verbindung der Formel (IIc) als eine wässrige Lösung.

5. Verfahren nach Anspruch 1 oder 2 zur Herstellung von 2-(1,1-Dicyanopropen-2-yl)-6-(2-fluoroethyl)-methylamino)-naphthalin (FDDNP), umfassend eine Behandlung eines an einen festen Träger gebundenen Vorläufers der Formel (Ih): 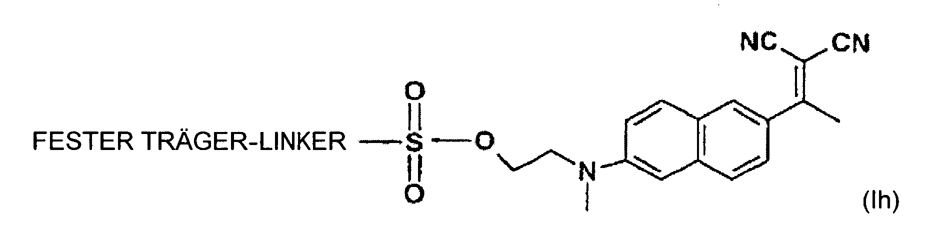 mit ¹⁸F, um den markierten Tracer der Formel (IIh) zu erzeugen. 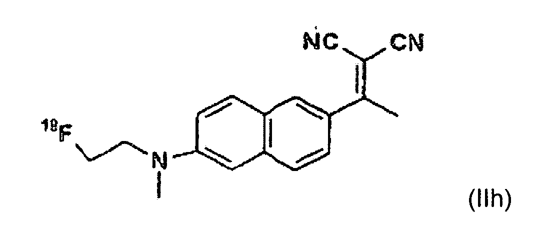 gegebenenfalls gefolgt von
(i) Entfernung von nicht umgesetztem ¹⁸F⁻, z. B. durch lonenaustauschchromatographie; und/oder
(ii) Entfernung von organischem Lösungsmittel; und/oder
(iii) Formulierung der resultierenden Verbindung der Formel (IIh) als eine wässrige Lösung.

6. Verfahren nach Anspruch 1 zur Herstellung eines ¹⁸F-markierten Tracers, umfassend eine Behandlung eines an einen festen Träger gebundenen Vorläufers der Formel (Id) 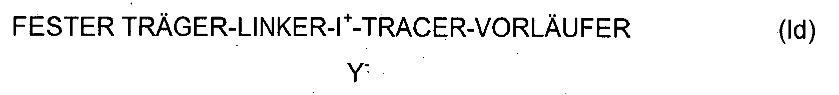 wobei Y⁻ ein Anion ist, bevorzugt ein Trifluoromethylsulphonat(Triflat)-Anion, mit ¹⁸F, um den markierten Tracer der Formel (IId) herzustellen
¹⁸F-TRACER (IId)
gefolgt von gegebenenfalls
(i) Entfernung von überschüssigem ¹⁸F⁻, z. B. durch lonenaustauschchromatographie; und/oder
(ii) Entfernung beliebiger Schutzgruppen; und/oder
(iii) Entfernung von organischem Lösungsmittel; und/oder
(iv) Formulierung der resultierenden Verbindung der Formel (IId) als eine wässrige Lösung.

7. Verfahren nach Anspruch 1 oder 6 zur Herstellung von 6-L-¹⁸F-Fluorodopa(¹⁸F-FDOPA), umfassend eine Behandlung eines an einen festen Träger gebundenen Vorläufers der Formel (Ie): 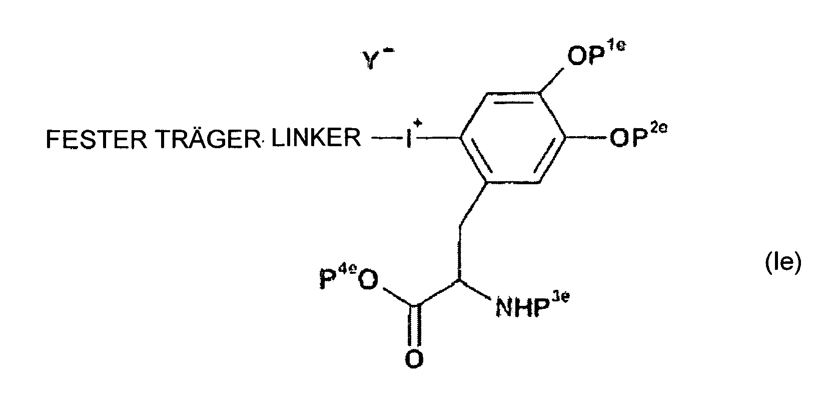 wobei P^{1e}, P^{2e}, P^{3e} und P^{4e} jeweils unabhängig für Wasserstoff oder eine Schutzgruppe stehen und Y⁻ für eine Anion steht, bevorzugt Trifluoromethylsulphonat(Triflat)-Anion;
mit ¹⁸F⁻, um den markierten Tracer der Formel (IIe) herzustellen 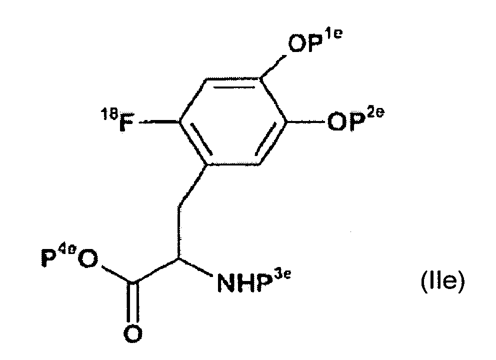 wobei P^{1e}, P^{2e}, P^{3e} und P^{4e} jeweils unabhängig für Wasserstoff oder eine Schutzgruppe stehen;
gegebenenfalls gefolgt von
(i) Entfernung von überschüssigem ¹⁸F⁻, z. B. durch lonenaustauschchromatographie; und/oder
(ii) Entfernung beliebiger Schutzgruppen; und/oder
(iii) Entfernung von organischem Lösungsmittel; und/oder
(iv) Formulierung der resultierenden Verbindung der Formel (IIe) als eine wässrige Lösung.

8. Verfahren zur Herstellung eines ¹⁸F-markierten Tracers der Formel (II), nach einem der Ansprüche 1 bis 7, zur Verwendung in PET.

9. Verbindung der Formel (Ib) 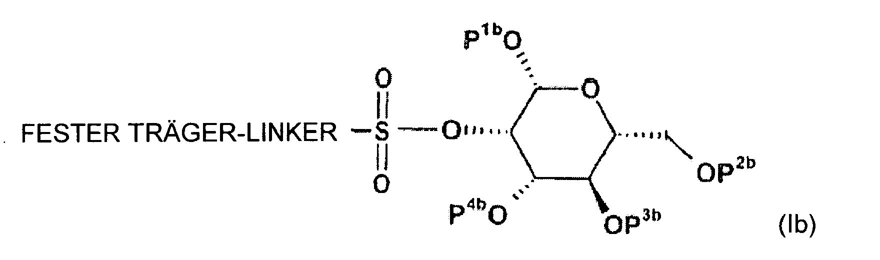 wobei P^{1b}, P^{2b}, P^{3b} und P^{4b} jeweils unabhängig für Wasserstoff oder eine Schutzgruppe stehen.

10. Verbindung der Formel (Ic): 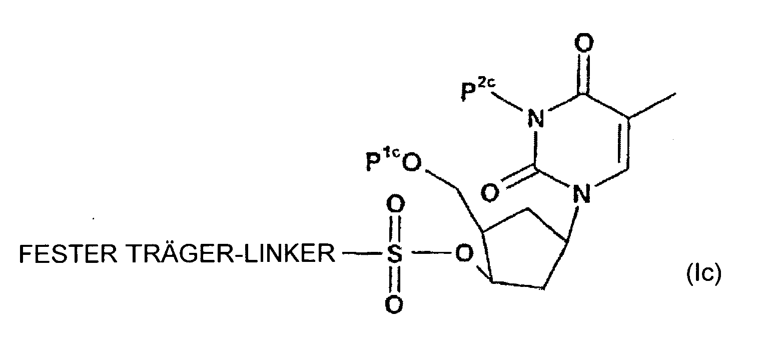 wobei P^{1c} und P^{2c} jeweils unabhängig für Wasserstoff oder eine Schutzgruppe stehen.

11. Verbindung der Formel (Ih):

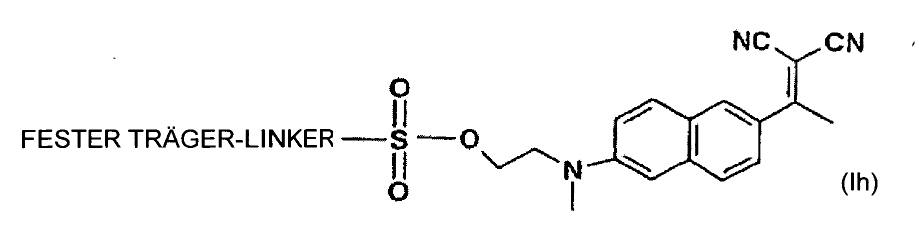

12. Verbindung der Formel (Ie): 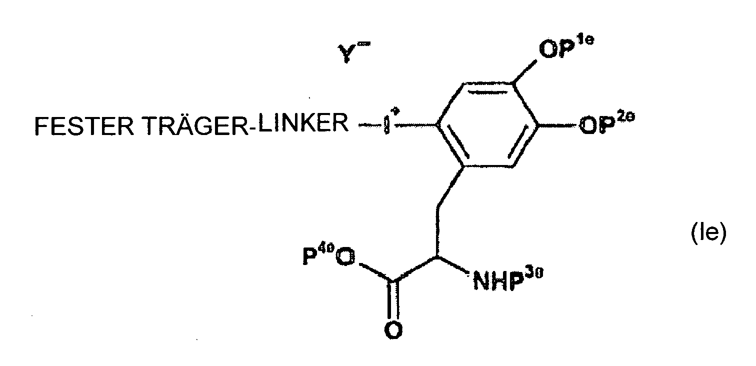 wobei P^{1e}, P^{2e}, P^{3e} und P^{4e} jeweils unabhängig für Wasserstoff oder eine Schutzgruppe stehen und Y⁻ für ein Anion steht, wie Triflat.

13. Radiopharmazeutisches Kit zur Herstellung eines ¹⁸F-markierten Tracers zur Verwendung in PET umfassend:
(i) ein Gefäß, enthaltend ein Verbindung der Formel (I) oder (Ia), (Ib), (Ic), (Id), (Ie) oder (Ih), wie definiert in einem der Ansprüche 1 bis 7; und
(ii) Mittel zum Eluieren des Gefäßes mit einer Quelle ¹⁸F;
(iii) eine Ionenaustauschkartusche zur Entfernung von überschüssigem ¹⁸F; und ggf.
(iv) eine Kartusche für eine Festphasen-Entschützung des resultierenden Produkts der Formeln (II) oder (IIb), (IIc), (IId), (IIe) oder (IIh), wie definiert in einem der Ansprüche 1 bis 7.

14. Kartusche für ein radiopharmazeutisches Kit zur Herstellung eines ¹⁸F-markierten Tracers zur Verwendung in PET umfassend:
(i) ein Gefäß, enthaltend eine Verbindung der Formel (I) oder (la), (Ib), (Ic), (Id), (Ie) oder (Ih), wie definiert in einem der Ansprüche 1 bis 7; und
(ii) Mittel zum Eluieren des Gefäßes mit einer Quelle von ¹⁸F⁻.

## Revendications

1. Procédé de production d'un traceur marqué par ¹⁸F qui comprend le traitement d'un précurseur lié par résine selon la formule (I)
SUPPORT SOLIDE-LIEUR-X-PRECURSEUR DE TRACEUR (I)
dans lequel X est un groupe qui favorise la substitution nucléophile au niveau d'un site spécifique sur le PRECURSEUR DE TRACEUR lié ;
avec du ¹⁸F- afin de produire le traceur marqué selon la formule (II)
¹⁸F- TRACEUR (II).

2. Procédé de production d'un traceur marqué par ¹⁸F selon la revendication 1, qui comprend le traitement d'un précurseur lié par résine selon la formule (la)
SUPPORT SOLIDE-LIEUR-SO₂-O-PRECURSEUR DE TRACEUR (la)
avec du ¹⁸F⁻ afin de produire le traceur marqué selon la formule (II)
¹⁸F-MARQUEUR (II)
suivi, en variante, par
(i) l'élimination de l'excès de ¹⁸F⁻, par exemple, par chromatographie par échange d'ion ; et/ou
(ii) l'élimination de groupes de protection quelconques ; et/ou
(iii) l'élimination de solvant organique ; et/ou
(iv) la formulation du composé résultant de formule (II) sous la forme d'une solution aqueuse.

3. Procédé selon la revendication 1 ou 2, afin d'assurer la production de 2-¹⁸F-fluoro-2-désoxy-D-glucose (¹⁸F-FDG) qui comprend le traitement d'un précurseur lié sur support solide selon la formule (Ib) : 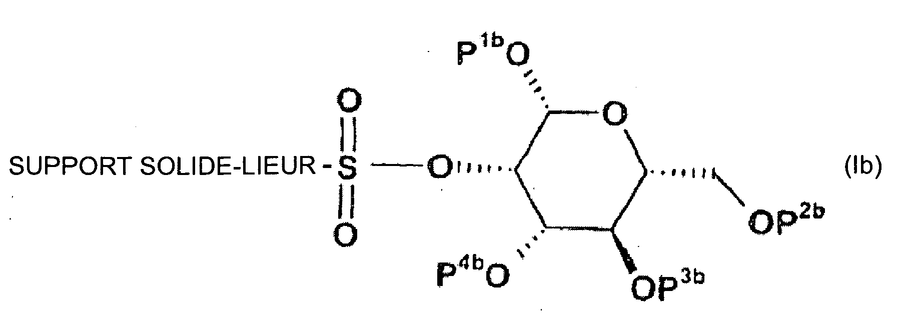 dans laquelle, P^{1b}, P^{2b}, P^{3b}, et P^{4b} sont chacun, de manière indépendante, de l'hydrogène ou un groupe de protection ;
avec du ¹⁸F- afin de produire le traceur marqué selon la formule (IIb) 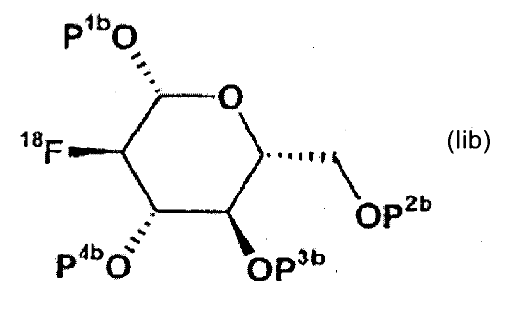 dans laquelle P^{1b}, P^{2b}, P^{3b}, et P^{4b} sont chacun, de manière indépendante, de l'hydrogène ou un groupe de protection ;
suivi, en variante, par
(i) l'élimination de l'excès de ¹⁸F⁻, par exemple, par chromatographie par échange d'ion ; et/ou
(ii) l'élimination de groupes de protection quelconques ; et/ou
(iii) l'élimination de solvant organique ; et/ou
(iv) la formulation du composé résultant de formule (II) sous la forme d'une solution aqueuse.

4. Procédé selon la revendication 1 ou 2, afin d'assurer la production de 3'-désoxy-3'-¹⁸F-fluorothymidine (¹⁸F-FLT) qui comprend le traitement d'un précurseur lié sur support solide selon la formule (Ic) : 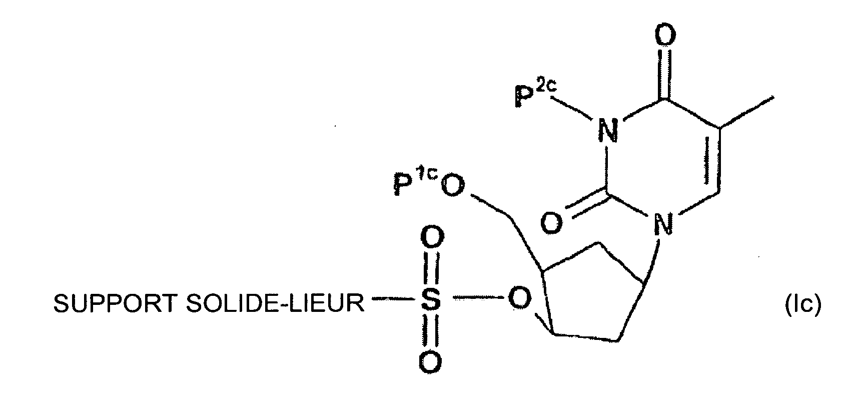 dans laquelle P^{1c} et P^{2c} sont chacun, de manière indépendante, de l'hydrogène ou un groupe de protection ; avec du ¹⁸F⁻ afin de produire le traceur marqué selon la formule (IIc) 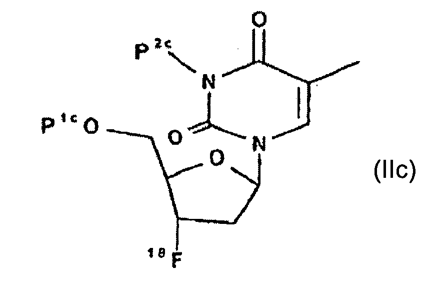 dans laquelle P^{1c} et P^{2c} sont chacun, de manière indépendante, de l'hydrogène ou un groupe de protection ;
suivi, en variante, par
(i) l'élimination de l'excès de ¹⁸F⁻, par exemple, par chromatographie par échange d'ion ; et/ou
(ii) l'élimination de groupes de protection quelconques ; et/ou
(iii) l'élimination de solvant organique ; et/ou
(iv) la formulation du composé résultant de formule (II) sous la forme d'une solution aqueuse.

5. Procédé selon la revendication 1 ou 2, pour la production de 2-(1,1-dicyanopropène-2-yl)-6-(2-fluoroéthyle)-méthylamino)-naphtalène (FDDNP) qui comprend le traitement d'un précurseur lié sur support solide selon la formule (Ih) : 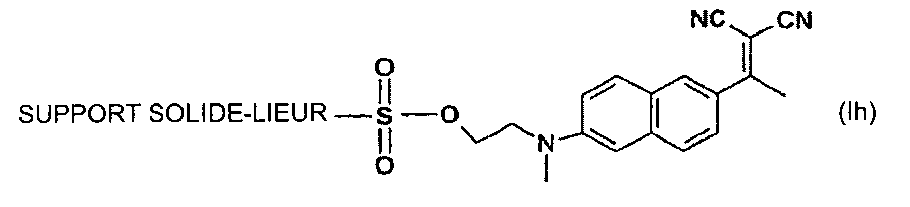 avec du ¹⁸F⁻ afin de produire le traceur marqué selon la formule (IIh) 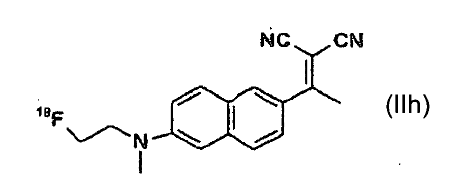 suivi en variante par
(i) l'élimination du ¹⁸F⁻ qui n'a pas réagi, par exemple, par chromatographie par échange d'ion ; et/ou
(ii) l'élimination de solvant organique ; et/ou
(iii) la formulation du composé résultant de formule (IIh) sous la forme d'une solution aqueuse.

6. Procédé selon la revendication 1, afin d'assurer la production d'un traceur marqué par ¹⁸F, qui comprend le traitement d'un précurseur lié sur support solide selon la formule (Id) 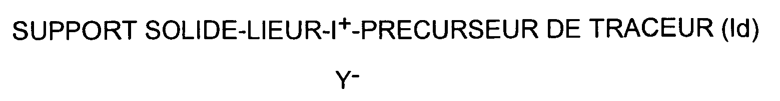 S Y⁻ est un anion, de préférence, un anion de trifluométhylsuphonate (triflate) avec du ¹⁸F⁻ afin de produire le traceur marqué selon la formule (IId)
¹⁸F-TRACEUR (IId)
suivi, en variante, par
(i) l'élimination de l'excès de ¹⁸F⁻, par exemple, par chromatographie par échange d'ion ; et/ou
(ii) l'élimination de groupes de protection quelconques ; et/ou
(iii) l'élimination de solvant organique ; et/ou
(iv) la formulation du composé résultant de formule (IId) sous la forme d'une solution aqueuse.

7. Procédé selon la revendication 1 ou 6, afin d'assurer la production de 6-L-¹⁸F-fluorodopa (¹⁸F-FDOPA) qui comprend le traitement d'un précurseur lié sur support solide selon la formule (Ie): 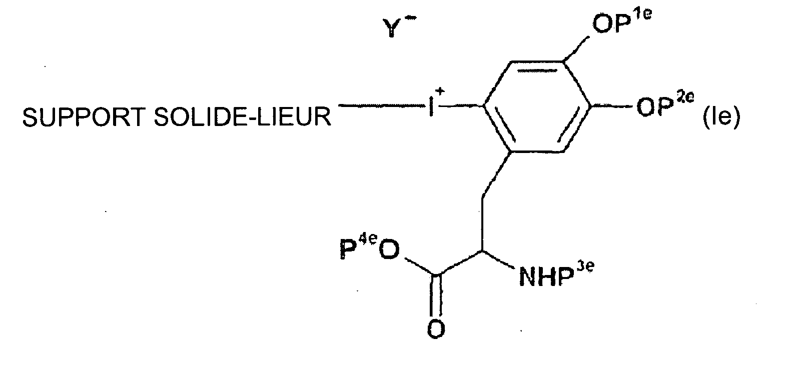 dans laquelle P^{1e}, P^{2e}, P^{3e}, et P^{4e} sont chacun, de manière indépendante, de l'hydrogène ou un groupe de protection Y⁻ est un anion, de préférence un anion de trifluorométhylsulphonate (triflate) ;
avec du ¹⁸F⁻ afin de produire le traceur marqué selon la formule (IIe) 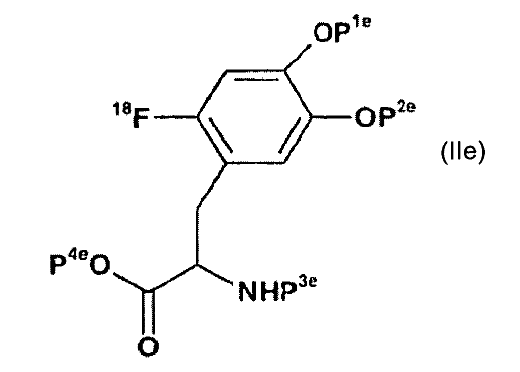 dans laquelle P^{1e}, P^{2e}, P^{3e}, et P^{4e} sont chacun, de manière indépendante, de l'hydrogène ou un groupe de protection ;
suivi, en variante, par
(i) l'élimination de l'excès de ¹⁸F⁻, par exemple, par chromatographie par échange d'ion ; et/ou
(ii) l'élimination de groupes de protection quelconques ; et/ou
(iii) l'élimination de solvant organique ; et/ou
(iv) la formulation du composé résultant de formule (IIe) sous la forme d'une solution aqueuse.

8. Procédé de fabrication d'un traceur marqué par ¹⁸F de formule (II), selon l'une quelconque des revendications 1 à 7, destiné à être utilisé en tomographie PET.

9. Composé selon la formule (Ib) 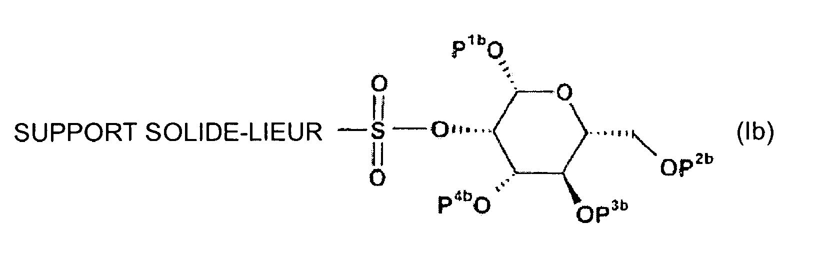 dans laquelle P^{1b}, P^{2b}, P^{3b}, et P^{4b} sont chacun, de manière indépendante, de l'hydrogène ou un groupe de protection.

10. Composé selon la formule (Ic) : 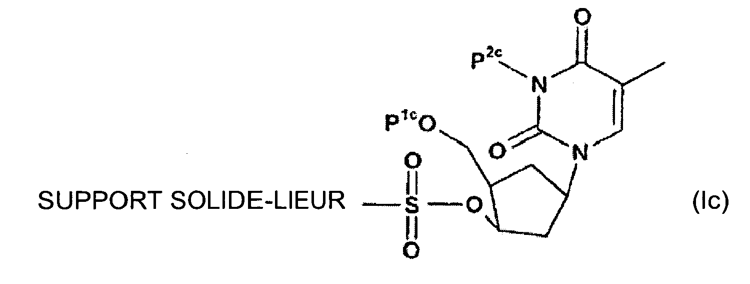 dans laquelle P^{1c} et P^{2c} sont chacun, de manière indépendante, de l'hydrogène ou un groupe de protection.

11. Composé selon la formule (Ih) :

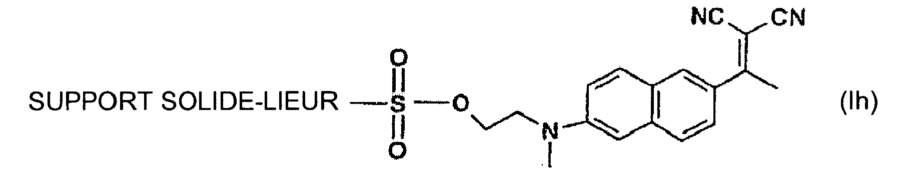

12. Composé selon la formule (Ie) : 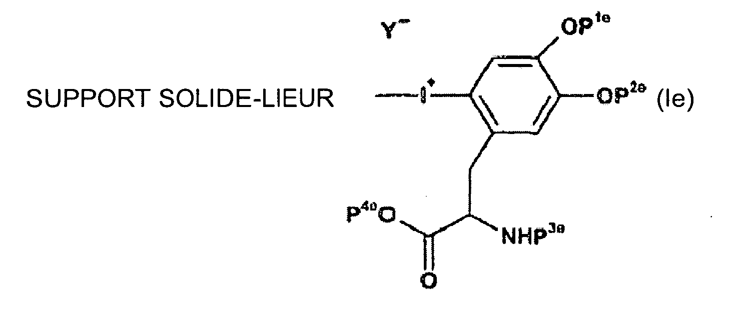 dans laquelle P^{1e}, P^{2e}, P^{3e}, et P^{4e} sont chacun, de manière indépendante, de l'hydrogène ou un groupe de protection est Y- est un anion tel que de triflate.

13. Kit radio-pharmaceutique de préparation d'un traceur destiné à être utilisé en tomographie PET qui comprend :
(i) une cuve contenant un composé de formule (I) ou (Ia), (Ib), (Ic), (Id), (Ie), ou (Ih) selon l'une quelconque des revendications 1 à 7 ; et
(ii) un moyen d'élution de la cuve avec une source de ¹⁸F⁻;
(iii) une cartouche d'échange d'ions afin d'assurer l'élimination de l'excès de ¹⁸F⁻ ; et, en variante,
(iv) une cartouche destinée à assurer la déprotection en phase solide du produit résultant de formule (II) ou (IIb), (IIc), (IId) (IIe) et (IIh) selon l'une quelconque des revendications 1 à 7.

14. Cartouche pour un kit radio-pharmaceutique de préparation d'un traceur marqué par ¹⁸F destiné à être utilisé en tomographie PET qui comprend :
(i) une cuve contenant un composé de formule (1) ou (Ia), (Ib), (Ic), (Id), (Ie) ou (Ih), selon l'une quelconque des revendications 1 à 7 ; et
(ii) un moyen d'élution de la cuve avec une source de ¹⁸F-.
